Europäisches Patentamt

European Patent Office

Office européen des brevets

(11) Publication number: **0 139 216**
**B1**

(12) **EUROPEAN PATENT SPECIFICATION**

(45) Date of publication of patent specification: **25.07.90**

(21) Application number: **84110996.0**

(22) Date of filing: **14.09.84**

(51) Int. Cl.⁵: **C 12 N 15/00, C 12 P 21/00, C 12 N 1/20** // (C12N1/20, C12R1:19)

(54) Adult T cell leukemia virus antigen peptide.

(30) Priority: **16.09.83 JP 170908/83**

(43) Date of publication of application:
**02.05.85 Bulletin 85/18**

(45) Publication of the grant of the patent:
**25.07.90 Bulletin 90/30**

(84) Designated Contracting States:
**BE DE FR GB**

(56) References cited:
**EP-A-0 105 465**
**EP-A-0 113 078**
**WO-A-84/04327**

**NATURE, vol. 294, 19th November 1981, pages 271-273, Macmillan Journals Ltd.; V.S. KALYANARAMAN et al.: "Antibodies in human sera reactive against an internal structural protein of human T-cell lymphoma virus"**

**PROCEEDINGS OF THE NATIONAL ACADEMY OF SCIENCES, USA, vol. 79, February 1982, pages 1291-1294; S. OROSZLAN et al.: "Primary structure analysis of the major internal protein p24 of human type C T-cell leukemia virus"**

(73) Proprietor: **KYOWA HAKKO KOGYO CO., LTD.**
**6-1, Ohte-Machi Itchome**
**Chiyoda-ku Tokyo (JP)**

(73) Proprietor: **Juridical Foundation Japanese Foundation for Cancer Research**
**37-1, Kami-Ikebukuro 1-chome Toshima-ku Tokyo (JP)**

(72) Inventor: **Taniguchi, Tadatsugu**
**A-207, 19, Mihogaoka**
**Ibaraki-shi Osaka-fu (JP)**
Inventor: **Yoshida, Mitsuaki**
**3-11-17, Jiyugaoka**
**Meguro-ku Tokyo (JP)**
Inventor: **Sugano, Haruo**
**4-8-13, Minami-Ogikubo**
**Suginami-ku Tokyo (JP)**
Inventor: **Sekine, Susumu**
**3-6-6, Asahi-machi**
**Machida-shi Tokyo (JP)**

(74) Representative: **Vossius & Partner**
**Siebertstrasse 4 P.O. Box 86 07 67**
**D-8000 München 86 (DE)**

(56) References cited:

PROCEEDINGS OF THE NATIONAL ACADEMY OF SCIENCES, USA, vol. 79, March 1982, pages 2031-2035; M. YOSHIDA et al.: "Isolation and characterization of retrovirus from cell lines of human adult T-cell leukemia and its implication in the disease"

PROCEEDINGS OF THE NATIONAL ACADEMY OF SCIENCES, USA, vol. 80, March 1983, pages 1574-1578; V. MANZARI et al.: "Human T-cell leukemia-lymphoma virus (HTLV): Cloning of an integrated defective provirus and flanking cellular sequences"

PROCEEDINGS OF THE NATIONAL ACADEMY OF SCIENCES, USA, vol. 80, June 1983, pages 3618-3622; M. SEIKI et al.: "Human adult T-cell leukemia virus: Complete nucleotide sequence of the provirus genome integrated in leukemia cell DNA"

**Description**

The present invention relates to an adult T cell leukemia virus antigen peptide encoded by an adult T cell leukemia virus *gag* gene, a recombinant plasmid bearing an inserted ATLV *gag* gene DNA fragment which codes for the peptide under the control of the tryptophan promoter, a microorganism containing the recombinant plasmid, and a method of producing adult T cell leukemia virus antigen peptide using the microorganism.

Adult T cell leukemia virus (hereinafter referred to as ATLV), which is a synonym of human T cell leukemia virus (HTLV), is a retrovirus isolated from patients with adult T cell leukemia (hereinafter referred to as ATL) [Hinuma et al.: Proc. Natl. Acad. Sci., USA, *78*, 6476—6480 (1981), Yoshida et al.: Proc. Natl. Acad. Sci., USA, *79*, 2031—2035 (1982)]. There are numerous reports that ATL patients have a poor prognosis and that efficacious treatment does not exist leading to a 50% mortality rate within 10 months.

In recent years, an antibody which reacts specifically with cultured MT—1 cells derived from ATL has been shown to exist in the serum of ATL patients (Hinuma et al., *supra*). The existence of this antibody has been confirmed subsequently in all ATL patients and the corresponding antigen is called ATL-associated antigen (hereinafter referred to as ATLA). It has been found that the antibody specific for ATLA (hereinafter referred to as Anti-ATLA antibody) exists in 25% of normal, healthy people in areas with a high incidence of ATL. It has also been shown that the distribution of cases possessing the anti-ATLA antibody corresponds to the regions with high ATL incidence. Furthermore, it has been shown that the retrovirus is generated within MT—1 cells, that ATLA is mainly an antigen of this retrovirus and that anti-ATLA antibody reacts with a structural protein of the virus, particularly the p24 protein. The existence of ATLV genome in the peripheral lymphocytes of patients has been established. ATLV has also been detected by culturing the lymphocytes of normal people who are positive to anti-ATLA antibody.

There is a very close correlation between ATL and ATLV, and ATLV is considered to be the causative virus of ATL. Though the route by which infection occurs is still unknown, it is considered that transfusion, maternal transmission and coitus transmission are the most likely routes. As 25% of healthy people in areas with a high incidence of ATL are anti-ATLA antibody positive, the likelihood of their being carriers of the disease is extremely high, which means that they must be avoided as blood transfusion donors.

Therefore early detection of the presence of anti-ATLA antibody will enable avoidance of transfusions from carriers and early ATL detection. At the present time, detection of anti-ATLA antibody is conducted using acetone fixed slides of MT—1 cells.

However, a more specific, simpler, faster method of anti-ATLA antibody detection and earlier ATL diagnosis are desirable. To this end, it has now been found that ATLV antigen peptide is accumulated and may be produced in good quantities by inserting the DNA fragment coding for the p24 structural protein within the gene for major antigen peptide in the ATLV genome, named the gag gene (group specific antigen), into a vector DNA by recombinant DNA technology to prepare a recombinant DNA and then culturing microorganisms hosting the recombinant DNA.

The amino acid sequence from N-terminal to the 25th amino acid in p24 has previously been reported by Oroszlan *et al.*: Proc. Natl. Acad. Sci., USA, *79*, 1291—1294 (1982). Furthermore, the entire DNA sequence of ATLV has been previously determined by the present inventors (European Patent Application No. 83 11 2261.9 filed December 7, 1983). By the present invention, a process for the production of the ATLV antigen peptide in a microorganism is provided.

Summary of the Invention

In accordance with the present invention a recombinant plasmid is constructed by inserting an ATLV *gag* gene DNA fragment under the control of a tryptophan product coding for adult T cell leukemia virus antigen peptide in a vector DNA. The recombinant plasmid includes a tryptophan promoter positioned upstream from the ATLV *gag* gene DNA fragment coding for adult T cell leukemia virus antigen peptide. The present invention also provides a method for producing adult T cell leukemia virus antigen peptide encoded by the ATLV *gag* gene by culturing, in a nutrient medium, a microorganism harboring a recombinant plasmid constructed by inserting an ATLV *gag* gene DNA fragment coding for adult T Cell leukemia virus antigen peptide under the control of a tryptophan promoter in a vector DNA plasmid, accumulating adult T cell leukemia virus antigen peptide encoded by the ATLV *gag* gene in the culture and recovering the peptide therefrom. The invention further provides microorganism transformants harboring a recombinant plasmid containing an ATLV *gag* gene DNA fragment coding for adult T cell leukemia virus antigen peptide. Finally, according to the composition of matter aspects of the invention, adult T cell leukemia virus antigen peptide encoded by the ATLV *gag* gene is provided.

Brief Description of the Drawings

In the accompanying drawings:
Fig. 1 illustrates the process for constructing plasmid pATK105 from plasmid pATK03;
Fig. 2 illustrates the process for constructing plasmids pTAG424A and pTAG424B from plasmid pATK105;
Fig. 3 illustrates the process for constructing plasmid pKYP—5;
Fig. 4 illustrates the process for constructing plasmid pKYP—10;

Fig. 5 illustrates the process for constructing plasmid pKYP—100; and
Fig. 6 illustrates the process for constructing plasmid pTrS3.

Description of the Invention

The present invention provides an ATLV antigen peptide encoded by the ATLV *gag* gene under the control tryptophan, a recombinant plasmid bearing an inserted ATLV *gag* gene DNA fragment coding for the peptide, a microorganism harboring the plasmid and a method for producing the ATLV antigen peptide encoded by the ATLV *gag* gene using the microorganism.

The recombinant plasmid according to the invention can be constructed by insertion of an ATLV *gag* gene DNA fragment coding for ATLV antigen peptide into a vector DNA using recombinant DNA techniques.

As structural proteins of the ATLV particle, p11, p14, p17, p24 and p45 are known. As the most commonly appearing among these is p24 and since it has a high reactivity with the serum of ATL patients, it is the most desirable as ATLV antigen peptide.

The plasmid pATK03 cloned by Seiki *et al.* [Seiki *et al.*: Proc. Natl. Acad. Sci., USA, *80*, 3613—3622 (1983)] can be used as a source of the DNA coding for ATLV antigen peptide. The ATLV genome consists of the LTR at both ends and at least 3 genes, namely, gag, pol, and env. It is assumed that p24, which is the virus antigen about which the most detailed research is being conducted, is the product of the gag gene as it is a core protein. Actually the DNA sequence coding for the amino acid sequence determined by Gallo *et al.* [Oroszlan *et al.*: Proc. Natl. Acad. Sci., USA, *79*, 1291—1294 (1982)] was detected in the gag gene. (Refer to the underlined section in Table 1 on page 15 and 16.) Thus, pATK03 is a clone which contains the entire gag gene and it can be used as a source of the DNA fragment coding for p24.

Any vector DNA can be utiilized, provided it is autonomously replicable in the intended host microorganism and the inserted DNA coding for ATLV antigen peptide is expressed within a microorganism. For this expression, the respective DNA-sequence is, dependant from the host, operatively linked to an expression control sequence selected from the group of the E. coli $\lambda$ promoter system, the E. coli lac system, the E. coli $\beta$-lactamase system, the E. coli trp-system, the E. coli lipoprotein promoter, yeast promoters and other eucaryotic expression control sequences. It is preferred to use a plasmid which includes a suitable promoter such as a tryptophan (trp) or lactose (lac) promoter downstream from which the subject DNA can be inserted. The downstream insertion site must be adjusted to have a suitable distance such as 6—18 base pairs, between the Shine-Dalgarno sequence (hereinafter referred to as SD sequence) and the translation initiation codon (ATG). One of the most suitable plasmids is pTrS3. Plasmid pTrS3 is constructed by the method described in Reference Example 3. Since pTrS3 vector has a distance (SD—ATG) of 13 base pairs between the SD sequence and the translation initiator codon (ATG) downstream from the tryptophan promoter and a foreign DNA can be inserted immediately after ATG, any gene which has the frame conforming with the ATG will be expressed directly and efficiently using this vector.

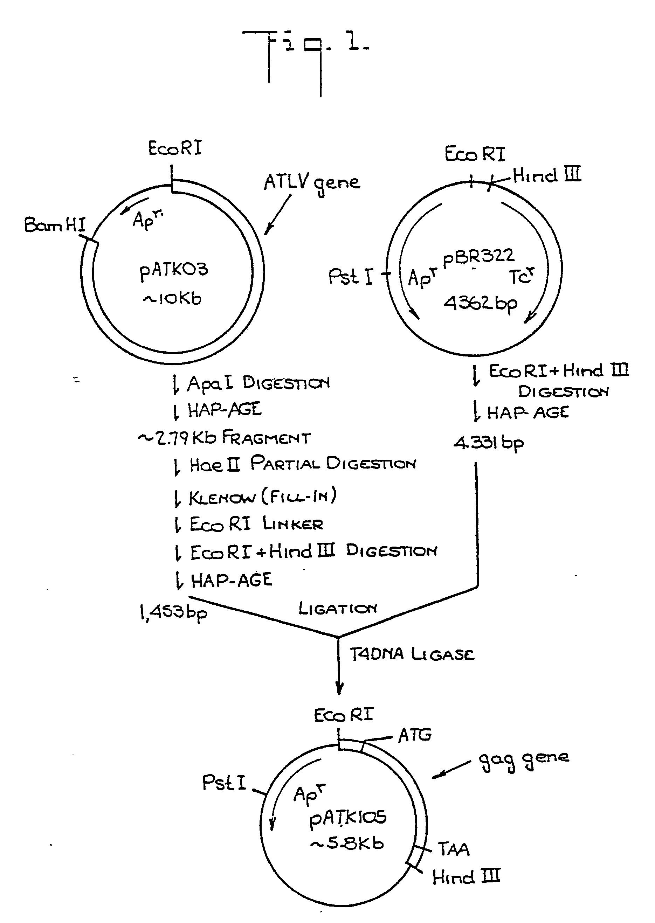

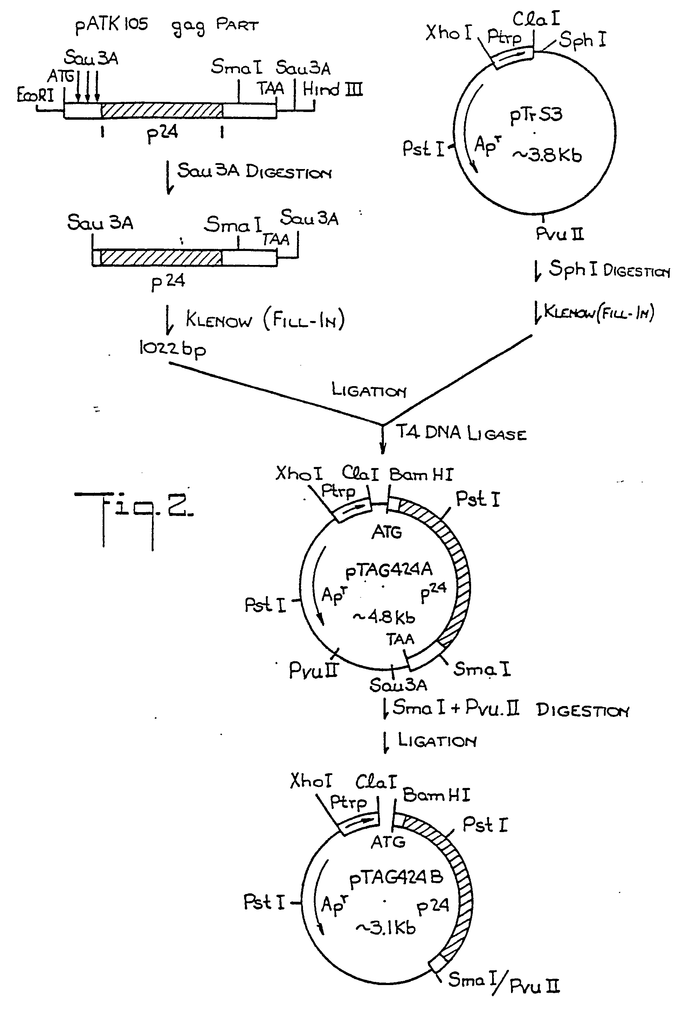

Recombination of a DNA coding for the ATLV antigen peptide such as the DNA coding for p24 from pATK03 and a vector DNA such as pTrS3 is generally carried out using recombinant DNA methods in which restriction enzymes are used to digest both DNAs followed by ligation using T4DNA ligase. Ligation may be conducted by a method employing fill-in reaction with DNA polymerase I·Klenow fragment or a method using DNA linker. In the case of pATK03 and pTrS3, as shown in Figs. 1 and 2, an EcoRI-HindIII digestion fragment containing the gag gene from pATK03 is subcloned in pBR322. The recombinant plasmid pTAG424A is constructed by inserting a fragment obtained by Sau3A digestion of the subcloned pATK105 into the SphI site of pTrS3. In addition, pTAG424B can be obtained by re-ligation after cleavage of pTAG424A with SmaI and PvuII.

The reaction conditions necessary for the above-described preparation of the recombinant plasmid are generally as follows. DNA digestion with restriction enzymes is normally carried out by 15 minutes—24 hours digestion of 0.1—100 µg of DNa, at 18—42°C, preferably 32—38°C, using 0.1—300 units, preferably 1—3 units, of restriction enzyme per 1 µg of DNA in 2—200 mM, preferably 10—40 mM Tris HCl (pH 6.0—9.5, preferably pH 7.0—8.0), 1—150 mM NaCl and 2—20 mM, preferably 5—10 mM $MgCl_2$. The reaction is terminated by heating at 55—75°C, preferably 63—70°C, for 5—30 minutes. The restriction enzymes may be inactivated by reagents such as phenol and diethyl pyrocarbonate. Synthetic oligonucleotides are prepared by the diethyl phosphate method [H. G. Khorana *et al.*: J. Mol. Biol., *72*, 209 (1972)], the phosphotriester method [R. Crea *et al.*: Proc. Natl. Acad. Sci., USA, *75*, 5765 (1978)] or the phosphite method [M. D. Matteucci *et al.*: J. Am. Chem. Soc. *103*, 3185 (1981)].

Phosphorylation of the synthetic oligonucleotides is conducted at 20—40°C, preferably 35—38°C for 5 minutes to 2 hours, using 0.1—100 units of T4 polynucleotide kinase in 2—200 mM, preferably 10—70 mM Tris-HCl (pH 6.0—9.5, preferably pH 7.0—8.0), 3—20 mM, preferably 4—10 mM $MgCl_2$ and 1—10 mM dithiothreitol. Ligation of DNA fragments is conducted at 1—37°C, preferably 3—20°C, for 15 minutes to 72 hours, preferably 2—20 hours using 0.1—10 units of T4DNA ligase in 2—200 mM, preferably 10—70 mM Tris-HCl (pH 6.0—9.5, preferably pH 7.0—8.0), 2—20 mM, preferably 5—10 mM $MgCl_2$, 0.1—10 mM, preferably 0.5—2 mM ATP and 1—50 mM, preferably 5—10 mM dithiothreitol.

Purification of the DNA fragments, recombinant plasmids, etc. is carried out by agarose gel electrophoresis.

ATLV antigen peptide is obtained by culturing a transformant obtained by introducing a recombinant plasmid such as pTAG424A or pTAG424B into a microorganism. It is desirable to use *Escherichia coli* as the host microorganism and HB101 or CSR 603 derived from the *Escherichia coli* K—12 strain is preferably used. Transformation is carried out in accordance with the method of S. N. Cohen *et al*: Proc. Nat·Acad. Sci., USA, *69*, 2110 (1972). Transformants containing pTAG424A or pTAG424B can be screened and obtained as ampicillin-resistant strains. Culturing of transformants is carried out using a medium and culturing conditions selected to be suitable for the particular strain employed as the host microorganism. Culturing is continued until recoverable quantities of the peptide are expressed by the transformant. Thereafter the peptide is recovered by standard methods.

Expression of the ATLV antigen peptide by the transformant is detected in accordance with the Maxicell method as Sancar *et al*.: J. Bact. *137*, 692—693 (1979). This involves culturing of the microorganism strain in M9-Casamino acid medium, followed by preferential chromosome inactivation using controlled ultra-violet irradiation. This process therefore permits in consequence the predominant expression of genes which are present within the plasmids, existing in multicopies. By using $^{35}$S-methionine as a marker, labelled proteins are detected by SDS polyacrylamide gel electrophoresis (Laemmli, Nature *227*, 680 (1970)]. The polypeptide detected in this way is ascertained to give specific immune agglutination with patient serum, which indicates that it has the properties of ATLV antigen peptide. Isolation of the plasmids from the microorganisms is carried out in accordance with the method of H. C. Birnboim *et al*.: Nucleic Acids Research *7*, 1513 (1979).

Certain specific embodiments of the invention are illustrated by the following representative examples.

Example 1

Insertion of the ATLV gag gene fragment into the expression vector pTrS3

(1) Subcloning of the gag gene of plasmid pATK03 (Fig. 1): 600 µg of pATK03 were dissolved in 2 ml of a solution consisting of 20 mM Tris-HCl (pH 7.5), 10 mM MgCl₂, 10 mM dithiothreitol and 100 mM NaCl. Then, 1,000 units of restriction enzyme ApaI (product of Boehringer Mannheim GmbH) were added and digestion was carried out at 37°C for 6 hours. DNA fragments were isolated by subjecting the digest to agarose gel electrophoresis. Hydroxyl apatite (product of Bio Rad Co., hereinafter referred to as HAP) was put into a groove formed directly in front of the desired fragment of 2.7 Kb on the gel. Electrophoresis was continued and when the subject band was adsorbed on the HAP, the DNA fragment-adsorbed HAP was collected with a pasteur pipette and put on a Sephadex G—50 column (1 cm × 20 cm) which had been equilibrated with 10 mM Tris-HCl (pH 7.5). DNA fragments were dissociated from the HAP with 0.5 M EDTA (pH 8.0) and elution was continued with 10 mM Tris-HCl (pH 7.5) to obtain the DNA fraction. After phenol and chloroform extraction of the fraction, a DNA fragment of 2.7 Kb was recovered by ethanol precipitation. Hereinafter, the method for recovery of a DNA fragment using agarose gel electrophoresis and HAP is referred to as AGE—HAP. Then, 40 µg of the DNA fragment were dissolved in 100 µl of a solution consisting of 20 mM Tris-HCl (pH 7.5), 10 mM MgCl₂ and 10 mM dithiothreitol. 10 units of restriction enzyme HaeII (product of Takara Shuzo Co., the restriction enzymes hereinafter are all products of Takara Shuzo Co., unless otherwise specified) were added and partial digestion of the DNA fragment was carried out at 37°C for 15 minutes. The reaction solution was subjected to AGE—HAP and 5 µg of DNA fragment of 1,795 base pairs were obtained. Then, 5 µg of the DNA fragment were dissolved in a solution consisting of 50 mM Tris-HCl (pH 7.8), 5 mM MgCl₂ and 1 mM dithiothreitol and 1 mM each of dATP, dTTP, dGTP and dCTP were added together with 15 units of *Escherichia coli* DNA polymerase I·Klenow fragment (Bethesda Research Laboratories Inc., hereinafter referred to as BRL). Fill-in reaction was performed at 15°C for 3 hours.

Separately, 4.8 µg of EcoRI linker (product of Takara Shuzo Co.) were dissolved in 30 µl of a solution of 50 mM Tris-HCl (pH 7.5), 10 mM MgCl₂, 5 mM dithiothreitol and 1 mM ATP. Five units of T4 polynucleotide kinase (product of Takara Shuzo Co.) were added and the mixture was subjected to phosphorylation reaction. Then, 2.4 µg of phosphorylated EcoRI linker were mixed with 5 µg of the fragment partially digested with HaeII described above. The mixture was then dissolved in 50 µl of a solution consisting of 20 mM Tris-HCl (pH 7.6), 10 mM MgCl₂, 10 mM dithiothreitol and 1 mM ATP, followed by addition of 2.5 units of T4DNA ligase (product of Takara Shuzo Co.). After ligation at 4°C for 16 hours, the whole DNA was recovered by ethanol precipitation.

Then, 4 µg of DNA fragment with attached EcoRI linker were dissolved in 100 µl of a solution consisting of 20 mM Tris-HCl (pH 7.5), 10 mM MgCl₂ and 60 mM NaCl. Five units each of EcoRI and HindIII were added and digestion reaction was carried out at 37°C for 2 hours. From this digest, 1.5 µg of EcoRI-HindIII digested fragment of 1,453 base pairs were obtained by AGE—HAP.

Separately, 5 µg of pBR322 (4.4 Kb) [Bolivar *et al*.: Gene, *2*, 95 (1977)] were dissolved in 100 µl of a solution consisting of 20 mM Tris-HCl, 10 mM MgCl₂ and 10 mM dithiothreitol. Then, 5 units each of EcoRI and HindIII were added and digestion was carried out at 37°C for 2 hours. 2.5 µg of EcoRI-HindIII fragment of about 4.3 Kb were recovered by AGE—HAP.

0.2 µg of the fragment and 0.35 µg of the EcoRI-HindIII fragment of 1,453 base pairs from pATK03 were dissolved in 50 µl of a solution consisting of 20 mM Tris-HCl (pH 7.6), 10 mM MgCl₂ 10 mM dithiothreitol and 1 mM ATP. Then, 2.5 units of T4DNA ligase were added and ligation reaction was carried out at 4°C for 16 hours.

Using this ligation solution, *Escherichia coli* K—12, HB101 strain [Bolivar *et al*.: Gene *2*, 75 (1977)] was transformed by conventional technique and an ampicillin-resistant (Ap$^R$) strain was obtained. The recombinant plasmid pATK105 shown in Fig. 1 was isolated from the strain by conventional technique. The structure of pATK105 was determined by AGE after digestion with the restriction endonucleases, EcoRl, HindlII and Pstl. *Escherichia coli* K—12, HB101 strain containing plasmid pATK105 has been deposited with the Fermentation Research Institute, Agency of Industrial Science and Technology (Fermentation Research Institute) as *Escherichia coli* EATK105, FERM BP—340.

pATK105 is a plasmid containing a sequence extending from the 18th base pair upstream from the ATG codon to the 139th base pair downstream from the terminator codon of the ATLV gag gene and is thus a suitable source of the gag gene for recombination.

(2) Cloning of the gag gene of pATK105 into pTrS3 vector (Fig. 2)

A DNA fragment containing the p24 coding region was cleaved from pATK105 and ligated directly next to the ATG codon of the expression vector pTrS3 in the following manner.

40 μg of pATK105 were dissolved in 200 μl of a solution consisting of 20 mM Tris-HCl (pH 7.5), 10 mM MgCl$_2$, 10 mM dithiothreitol and 50 mM NaCl. Then, 50 units of Sau3A were added and digestion was carried out at 37°C for 3 hours. 25 μg of Sau3A DNA fragment of 1,022 base pairs were obtained from this reaction solution by AGE—HAP. Then, about 2 μg of the DNA fragment were dissolved in 30 μl of a solution consisting of 50 mM Tris-HCl (pH 7.8), 5 mM MgCl$_2$ and 1 mM dithiothreitol, followed by addition of 1 mM each of dATP, dTTP, dGTP and dCTP, together with 3 units of *Escherichia coli* DNA polymerase I·Klenow fragment. Fill-in reaction was carried out at 15°C for 3 hours.

Separately, 10 μg of pTrS3 (3.8 Kb) obtained by the reference example method were dissolved in 100 μl of a solution consisting of 20 mM Tris-HCl (pH 7.5), 10 mM MgCl$_2$, 10 mM dithiothreitol and 150 mM NaCl. Then, 10 units of Sphl (product of BRL) were added and digestion reaction was carried out at 37°C for 3 hours. The DNA recovered by ethanol precipitation was dissolved in 100 μl of a solution consisting of 50 mM Tris-HCl (pH 7.8), 5 mM MgCl$_2$, 10 mM dithiothreitol and 1 mM each of dATP, dTTP, dGTP and dCTP. Then, 10 units of *Escherichia coli* DNA polymerase I·Klenow fragment were added and trimming, reaction was carried out at 15°C for 3 hours. About 1 μg of a DNA fragment of 3.8 Kb was recovered by AGE—HAP.

Thereafter, 0.1 μg of the DNA fragment and 0.4 μg of the Sau3A DNA fragment prepared by the above-described fill-in reaction were dissolved in 50 μl of a solution consisting of 20 mM Tris-HCl (pH 7.6), 10 mM MgCl$_2$, 10 mM dithiothreitol and 1 mM ATP. 2.5 units of T4DNA ligase were added and ligation reaction was carried out at 4°C for 16 hours. *Escherichia coli* K—12, KB101 strain was transformed by conventional technique using this ligation solution and a Ap$^R$ strain was obtained. Plasmids were isolated from the strain by conventional technique and the recombinant plasmid pTAG424A was recovered. The structure of pTAG424A was confirmed by AGE after digestion with Xhol, Pstl, Pvull, Sau3A, Clal, Smal and BamHl. The structure between SD—ATG was investigated by the method of Maxam and Gilbert, Proc. Natl. Acad. Sci., USA, *74*, 560 (1977). It was thus determined that pTAG424A codes for a polypeptide consisting of a total of 317 amino acids, wherein 17 amino acids and 85 amino acids derived from the gag gene are respectively attached to the N-terminal and C-terminal of p24. It was also confirmed that the base sequence between SD—ATG is

*AAGG*TATCGATAAGCT*ATG.*
SD

*Escherichia coli* K—12, HB101 strain which contains plasmid pTAG424A has been deposited with the Fermentation Research Institute as *Escherichia coli* ETAG424A, FERM BP—341. A derivative can be constructed from pTAG424A by removing a DNA sequence which corresponds to an amino acid sequence other than p24 using appropriate restriction enzymes, exonuclease BAL31 and *Escherichia coli* DNA polymerase I.

TABLE 1

ATLV Gag Gene and the Sequence Around It

```
                                              LTR
         10        20        30     ↓   40        50          60
CCTATAGCACTCTCAGGAGAGAAATTTAGTACACAGTTGGGGGCTCGTCCGGGATACGAG


   HaeII                    gag                       HaeII
    ↓           70        80 ↓      90       100        ↓ 110        120
CGCCCCTTTATTCCCTAGGCAATGGGCCAAATCTTTTCCCGTAGCGCTAGCCCTATTCCG
                    Met Gly Gln Ile Phe Ser Arg Ser Ala Ser Pro Ile Pro


         130       140       150       160       170       180
CGACCGCCCCGGGGGCTGGCCGCTCATCACTGGCTTAACTTCCTCCAGGCGGCATATCGC
Arg Pro Pro Arg Gly Leu Ala Ala His His Trp Leu Asn Phe Leu Gln Ala Ala Tyr Arg


         190       200       210       220       230       240
CTAGAACCCGGTCCCTCCAGTTACGATTTCCACCAGTTAAAAAAATTTCTTAAAATAGCT
Leu Glu Pro Gly Pro Ser Ser Tyr Asp Phe His Gln Leu Lys Lys Phe Leu Lys Ile Ala


                   ·Sau3A
         250      ↓ 260      270       280       290       300
TTAGAAACACCGGCTCGGATCTGTCCCATTAACTACTCCCTCCTAGCCAGCCTACTCCCA
Leu Glu Thr Pro Ala Arg Ile Cys Pro Ile Asn Tyr Ser Leu Leu Ala Ser Leu Leu Pro


                                                           Sau3A
          310       320       330       340       350      └ 360
AAAGGATACCCCGGCCGGGTGAATGAAATTTTACACATACTCATCCAAACCCAAGCCCAG
Lys Gly Tyr Pro Gly Arg Val Asn Glu Ile Leu His Ile Leu Ile Gln Thr Gln Ala Gln


         370       380       390       400       410       420
ATCCCGTCCCGTCCCGCGCCACCGCCGCCGTCATCCCCCACCCACGACCCCCCGGATTCT
Ile Pro Ser Arg Pro Ala Pro Pro Pro Ser Ser Pro Thr His Asp Pro Pro Asp Ser


Sau3A                                                p24
  ↓      430       440       450       460       470 ─┐ 480
GATCCACAAATCCCCCCTCCCTATGTTGAGCCTACGGCCCCCCAAGTCCTTCCAGTCATG
Asp Pro Gln Ile Pro Pro Pro Tyr Val Glu Pro Thr Ala Pro Gln Val Leu Pro Val Met


         490       500       510       520       530       540
CATCCACATGGTGCTCCTCCTAACCATCGCCCATGGCAAATGAAAGACCTACAGGCCATT
His Pro His Gly Ala Pro Pro Asn His Arg Pro Trp Gln Met Lys Asp Leu Gln Ala Ile


         550       560       570       580       590       600
AAGCAAGAAGTCTCCCAAGCAGCCCCTGGGAGCCCCCAGTTTATGCAGACCATCCGGCTT
Lys Gln Glu Val Ser Gln Ala Ala Pro Gly Ser Pro Gln Phe Met Gln Thr Ile Arg Leu


                                                 PstI
         610       620       630       640       650 |     660
GCGGTGCAGCAGTTTGACCCCACTGCCAAAGACCTCCAAGACCTCCTGCAGTACCTTTGC
Ala Val Gln Gln Phe Asp Pro Thr Ala Lys Asp Leu Gln Asp Leu Leu Gln Tyr Leu Cys


         670       680       690       700       710       720
TCCTCCCTCGTGGCTTCCCTCCATCACCAGCAGCTAGATAGCCTTATATCAGAGGCCGAA
Ser Ser Leu Val Ala Ser Leu His His Gln Gln Leu Asp Ser Leu Ile Ser Glu Ala Glu


         730       740       750       760       770       780
ACCCGAGGTATTACAGGTTATAACCCATTAGCCGGTCCCCTCCGTGTCCAAGCCAACAAT
Thr Arg Gly Ile Thr Gly Tyr Asn Pro Leu Ala Gly Pro Leu Arg Val Gln Ala Asn Asn


         790       800       810       820       830
CCACAACAACAAGGATTAAGGCGAGAATACCAGCAACTCTGGCTCGCCGCCTTCGCCGCC
Pro Gln Gln Gln Gly Leu Arg Arg Glu Tyr Gln Gln Leu Trp Leu Ala Ala Phe Ala Ala
```

# EP 0 139 216 B1

## TABLE 1
### ATLV Gag Gene and the Sequence Around It

```
          850       860       870       880       890       900
CTGCCGGGGGAGTGCCAAAGACCCTTCCTGGGCCTCTATCCTCCAAGGCCTGGAGGAGCCT
Leu Pro Gly Ser Ala Lys Asp Pro Ser Trp Ala Ser Ile Leu Gln Gly Leu Glu Glu Pro

          910       920       930       940       950       960
TACCACGCCTTCGTAGAACGCCTCAACATAGCTCTTGACAATGGGCTGCCAGAAGGCACG
Tyr His Ala Phe Val Glu Arg Leu Asn Ile Ala Leu Asp Asn Gly Leu Pro Glu Gly Thr

          970       980       990      1000      1010      1020
CCCAAAGACCCCATCTTACGTTCCTTAGCCTACTCCAATGCAAACAAAGAATGCCAAAAA
Pro Lys Asp Pro Ile Leu Arg Ser Leu Ala Tyr Ser Asn Ala Asn Lys Glu Cys Gln Lys

         1030        1040      1050      1060      1070      1080
TTACTACAGGCCCGAGGACACACTAATAGCCCTCTAGGAGATATGTTGCGGGCTTGTCAG
Leu Leu Gln Ala Arg Gly His Thr Asn Ser Pro Leu Gly Asp Met Leu Arg Ala Cys Gln

                                        p24
         1090      1100      1110      ↓ 1120      1130      1140
ACCTGGACCCCCAAAGACAAAACCAAAGTGTTAGTTGTCCAGCCTAAAAAACCCCCCCCA
Thr Trp Thr Pro Lys Asp Lys Thr Lys Val Leu Val Val Gln Pro Lys Lys Pro Pro Pro

         1150      1160      1170      1180      1190      1200
AATCAGCCGTGCTTCCGGTGCGGGAAAGCAGGCCACTGGAGTCGGGACTGCACTCAGCCT
Asn Gln Pro Cys Phe Arg Cys Gly Lys Ala Gly His Trp Ser Arg Asp Cys Thr Gln Pro

              Sma
         1210 ↓    1220      1230      1240      1250      1260
CGTCCCCCCCCCGGGCCATGCCCCCTATGTCAAGACCCAACTCACTGGAAGCGAGACTGC
Arg Pro Pro Pro Gly Pro Cys Pro Leu Cys Gln Asp Pro Thr His Trp Lys Arg Asp Cys

         1270      1280      1290      1300      1310      1320
CCCCGCCTAAAGCCCACTATCCCAGAACCAGAGCCAGAGGAAGATGCCCTCCTATTAGAC
Pro Arg Leu Lys Pro Thr Ile Pro Glu Pro Glu Pro Glu Glu Asp Ala Leu Leu Leu Asp

                                                      gag
         1330      1340      1350      1360      1370┐    1380
CTCCCCGCTGACATCCCACACCCAAAAAAACTCCATAGGGGGGGGAGGTTTAACCTCCCCCC
Leu Pro Ala Asp Ile Pro His Pro Lys Asn Ser Ile Gly Gly Glu Val

         1390      1400      1410      1420      1430      1440
CCACATTACAGCAAGTCCTTCCTAACCAAGACCCAGCATCTATTCTGCCAGTTATACCGT

         1450      1460      1470      1480      1490      1500
TAGATCCCGCCCGTCGGCCCGTAATTAAAGCCCAGGTTGACACCCAGACCAGCCACCCAA

       HindIII
    1510┐    1520      1530
AGACTATCGAAGCTTTACTAGATACAGGAG
```

## Example 2
### Construction of pTAG424B from pTAG424A

To construct plasmid pTAG424B, 5 μg of pTAG424A (about 4.8 Kb) obtained in Example 1 were dissolved in a solution consisting of 20 mM Tris-HCl (pH 7.5), 10 mM $MgCl_2$ and 50 mM NaCl. Then, 10 units each of SmaI and PvuII were added and digestion reaction was carried out at 37°C for 3 hours. From this reaction solution, 2 μg of SmaI-and PvuII-cleaved DNA fragment of 3.1 Kb were recovered by AGE—HAP. Then, 0.2 μg of the DNA fragment were dissolved in 50 μl of a solution consisting of 20 mM Tris-HCl (pH 7.6), 10 mM $MgCl_2$, 10 mM dithiothreitol and 1 mM ATP. After addition of 2.5 units of T4DNA ligase, ligation reaction was carried out at 4°C for 16 hours. The reaction solution was used to transform *Escherichia coli*

8

K—12, HB101 strain by conventional technique to obtain an Ap$^R$ strain. Plasmids were isolated from this strain by conventional technique to obtain the recombinant plasmid, pTAG424B, shown in Fig. 2. The structure of pTAG424B was confirmed by AGE after digestion with BamHI, PstI, SmaI and PvuII. The polypeptide encoded by pTAG424B compared with that encoded by pTAG424A is shorter by a section corresponding to 52 amino acids at the C-terminal and has a section corresponding to 6 amino acids derived from pBR322.

Escherichia coli K—12, HB101 strain containing plasmid pTAG424B has been deposited with the Fermentation Research Institute as Escherichia coli ETAG424B, FERM BP—342.

Example 3

Identification of the peptides encoded by pTAG424A and pTAG424B was carried out by the Maxi-cell method of Sancar et al.: J. Bact., 137, 692—693 (1979) as described below.

pTAG424A and pTAG424B, respectively, were used to transform Escherichia coli CSR603 strain (recA1, uvrA6, phr—1) by conventional technique. Ap$^R$ strains, namely a strain containing pTAG424A and a strain containing pTAG424B, were obtained. These strains and also the strain containing pTrS3 (FERM P—6735) were inoculated in 10 ml of an M9-Casamino acid medium (pH 7.4) consisting of 0.6% $Na_2HPO_4$, 0.3% $KH_2PO4$, 0.05% NaCl, 0.1% $NH_4Cl$, 2 mM $MgSO_4$, 0.2% glucose, 0.1 mM $CaCl_2$ and 0.5% Casamino acid and further containing 50 µg/ml ampicillin (Ap) and 50 µg/ml tryptophan. Culturing was carried out with shaking at 37°C. At $OD_{660}$ 0.2, the culture fluid was transferred to a watch glass containing a stirring bar. Agitation was continued with the stirrer during 10 seconds of irradiation at 90 cm directly under a UV lamp (15W). The culture fluid was then transferred to a large test tube (50 ml) and shaking was continued at 37°C for 1 hour. Thereafter, 100 µg/ml (final concentration) D-cycloserine were added and culturing was carried out at 37°C for 16 hours. The culture fluid was then centrifuged (3300 rpm, 10 minutes) and cells were harvested. The cells were washed twice with sulfur-free M9 medium (M9-Casamino acid medium containing 2 mM $MgCl_2$ from which Casamino acid and $MgSO_4$, were removed). The cells were then suspended in 1 ml of sulfur-free M9 medium containing 50 µg/ml Ap and 10 µg/ml indole acrylic acid (IAA), and the suspension was shaken at 37°C for 1 hour. $^{35}$S-methionine (~1000 Ci/m mole, product of Amersham Co.) was added to give 20 µCi/ml and culturing was carried out with shaking at 37°C for 1 hour to incorporate the marker. The cells were collected by centrifugation (3,300 rpm, 10 minutes) and suspended in 100 µl of Laemmli's sample buffer [Laemmli, Nature, 227, 680 (1970)]. The suspension was then heated at 100°C for 5 minutes to lyse the cells. Then, 20 µl of the lysate were subjected to SDS-polyacrylamide gel electrophoresis using the above referenced method of Laemmli.

Following the electrophoresis, the $^{35}$S-labeled peptide was detected by fluorography (EN$^3$ HANCE, product of New England Nuclear Co.). As a result, only a mature β-lactamase band was detected in the pTrS3 vector. However, in pTAG424A, a peptide band with a molecular weight of about 35,000, and in pTAG424B, a peptide band with a molecular weight of about 30,000, was detected in addition to the β-lactamase band. The molecular weights exhibited by the pTAG424A and pTAG424B bands are almost identical to the molecular weights of the polypeptides estimated from the base sequence of the gag gene DNA fragment.

Example 4

In this example, the cells labeled in Example 3 were solubilized and extracts were subjected to immunoprecipitation reaction with the serum of ATL patients and healthy people by the method of Richert et al.: J. Virol. 31, 695—706 (1979). By analyzing the sediment by SDS-polyacrylamide gel, it was determined that the peptides encoded by pTAG424A and pTAG424B do not react at all with the serum of healthy people but react specifically with the serum of ATL patients. This indicates that the peptides encoded by pTAG424A and pTAG424B have the properties of ATLV antigen and are suitable for the detection of anti-ATL antibody.

Reference Example 1

Construction of a plasmid vector pKYP10 having a trp promoter
(a) Purification of tryptophan operon transducing phage DNA

A λtrp phage, λcl857trpED10 (referred to as λtrp ED hereinafter) [G. F. Miozzari et al.: J. Bacteriol. 133, 1457 (1978)] was lysogenized in Escherichia coli JA 194 strain (F⁻, λ⁻, r⁻, m⁻, ΔtrpE5, leu6) [J. Carbon et al.: Recombinant Molecules p.355 (1977), Raven Press]. The resultant lysogenic strain, JA 194 (trpED) was cultured at 42°C for 30 minutes to induce λtrpED phages and prepare λphage lysate. The λphages were purified from the λphage lysate by the cesium chloride equilibrium density gradient centrifugation method of Yamakawa et al: "Chemicals of Nucleic Acids I", Tokyo Kagaku Dojin pp.54—61 (1974). The λphages were further purified by phenol treatment and chloroform treatment according to the method of Yamakawa et al.: "Chemicals of Nucleic Acids I" Tokyo Kagaku Dojin, p.62—65 (1974).

(b) Cloning of the trp promoter into a plasmid

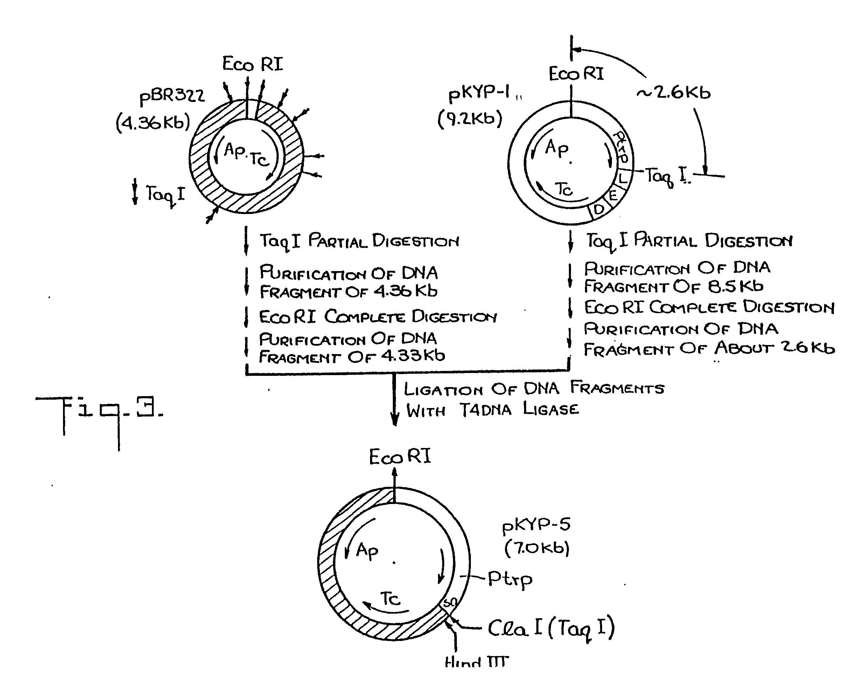

To clone the trp operon from λtrpED phage DNA, 8 µg of λtrpED DNA were digested at 37°C for 2 hours with 16 units of EcoRI and 16 units of HindIII in 20 mM Tris-HCl (pH 7.5), 75 mM NaCl, 10 mM $MgCl_2$ and 5 mM dithiothreitol. Separately, 1 µg of plasmid pBR325 DNA was digested with 2 units of EcoRI and 2 units

of HindIII by the same method as mentioned above (final volume: 30 μl). The reactions were stopped by heating at 65°C for 5 minutes. Then, 15 μl each of the digests were mixed and 500 μM (final concentration) ATP and 5 units of T4DNA ligase were added. The mixture was allowed to react at 4°C for 18 hours. *Escherichia coli* C600 SF strain, [Cameron *et al.*: Proc. Natl. Acad. Sci. *72*, 3416 (1975)] was transformed with the obtained plasmid mixture by the method of S. N. Cohen *et al.*: Proc. Natl. Acad. Sci. *69*, 2110 (1972) and transformants which were resistant to ampicillin (Ap$^R$), resistant to tetracycline (Tc$^R$) and sensitive to chloramphenicol (Cm$^S$) were selected. Plasmid DNAs were isolated from the transformants of *Escherichia coli*. One of the plasmid DNAs was named pKYP—1. This plasmid was digested with TaqI and EcoRI and the digest was subjected to purification by agarose gel electrophoresis to obtain a 2.6 Kb DNA fragment containing the tryptophan promoter and SD sequence. The 2.6 Kb DNA fragment was cloned in a known vector, pBR322, by the method as illustrated in Fig. 3. That is, 8 μg of pBR322 were digested at 45°C for 60 minutes with 2 units of TaqI in 100 μl of a reaction mixture comprising 10 mM Tris-HCl (pH 8.4), 6 mM MgCl$_2$, 100 mM NaCl and 6 mM 2-mercaptoethanol. After partial digestion with TaqI, the digest was subjected to low-gelling-temperature agarose gel : electrophoresis [Lars Wieslander: Analytical Biochemistry *98*, 305 (1979)] to obtain a purified 4.36 Kb DNA fragment. About 1.5 μg of the DNA fragment were completely digested at 37°C for 3 hours with 3 units of EcoRI. About 1.0 μg of an about 4.33 kb DNA fragment was recovered by low-gelling-temperature agarose gel electrophoresis in a similar manner as above.

Then, 12 μg of pKYP—1 DNA were partially digested with 3 units of TaqI. The digest was subjected to low-gelling-temperature agarose gel electrophoresis to obtain about 2 μg of a purified 8.5 Kb DNA fragment and the DNA was then completely digested with EcoRI by the same procedure as above to obtain about 0.5 μg of a purified 2.6 Kb DNA fragment. Then, 0.4 μg of the 4.33 Kb DNA of pBR322 and 0.25 μg of the 2.6 Kb DNA fragment of pKYP—1 thus obtained were added to 20 μl of a reaction solution consisting of 20 mM Tris-HCl (pH 7.6), 10 mM MgCl$_2$ and 10 mM dithiothreitol. Then, 0.5 mM ATP and 4 units of T4DNA ligase were added to the mixture and reaction was carried out at 4°C for 18 hours. *Escherichia coli* C600 SF8 strain was transformed with the recombinant plasmid DNA obtained as above. The plasmids in the transformant having Ap$^R$ and Tc$^R$ were isolated. The plasmid DNA was disgested with 6 restriction endonucleases, EcoRI, HindII, ClaI (product of Boehringer Mannheim GmbH), HpaI, HincII and BamHI to analyse the structure of the plasmid. The plasmid was named pKYP—5.

(c) *Preparation of a portable promoter from the trp promoter*

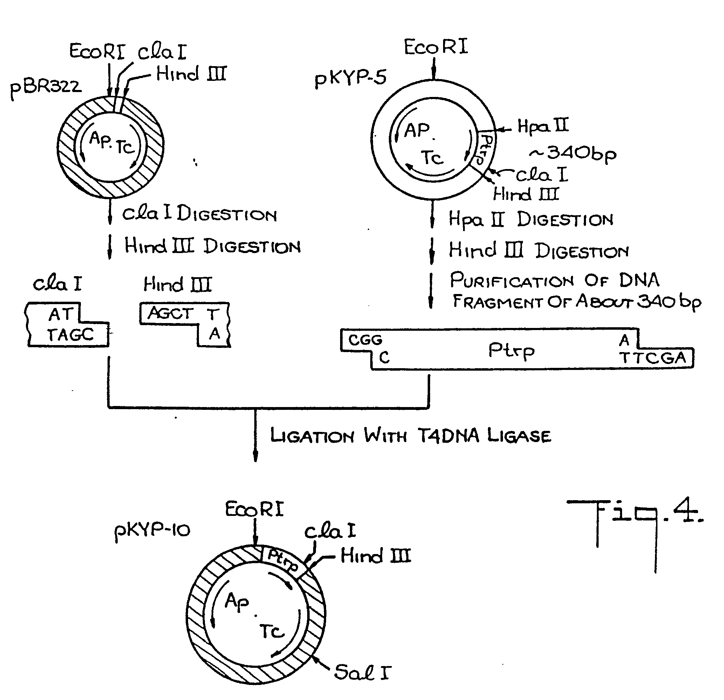

The plasmid vector pKYP—5 is applicable as a DNA introducing vector since it has a ClaI site and a HindIII site on the DNA of 1 to 20 base pairs downstream from the SD sequence. However, another ClaI site is present in pKYP—5 DNA besides the ClaI site immediately after the SD sequence and the fragment with the trp promoter obtained by cutting pKYP—5 DNA with EcoRI and HindIII is a little too large, i.e. 2.65 Kb. For convenience of use, pKYP—5 was improved by the process as illustrated in Fig. 4 to obtain a plasmid having a DNA fragment containing a shorter tryptophan promoter. That is, pKYP—5 DNA was digested with HpaII and HindIII and the digest was purified to obtain a DNA fragment of about 340 bp (base pairs). The fragment was inserted into the pBR322 digested with ClaI and HindIII as illustrated in Fig. 4 to obtain pKYP—10. The structure of pKYP—10 was determined by agarose gel electrophoresis after digestion with EcoRI, ClaI, HindIII and HpaI.

Reference Example 2

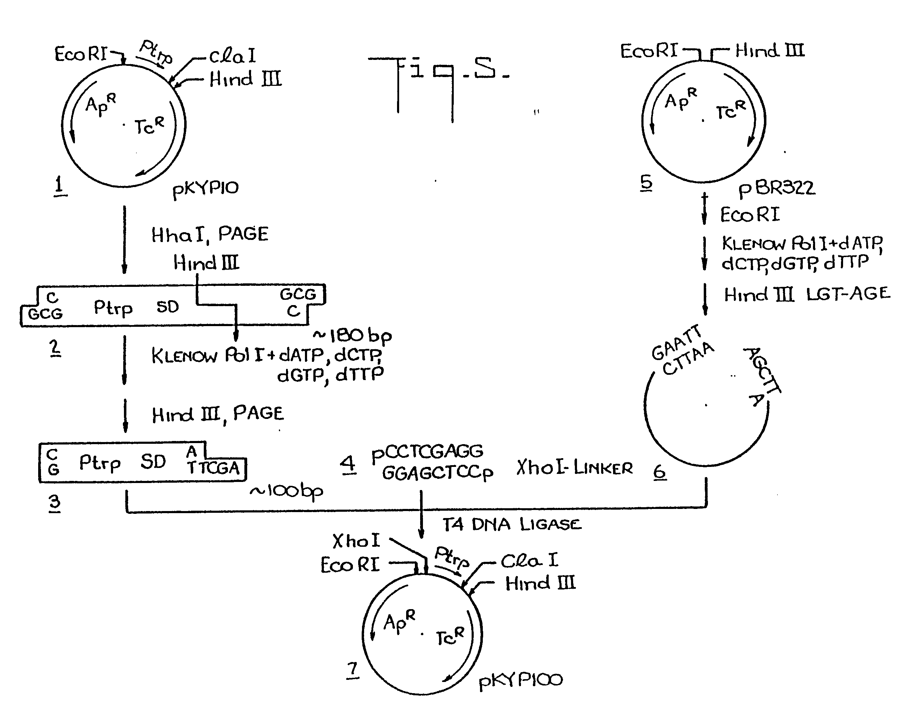

Construction of pKYP100 (refer to Fig. 5): To construct plasmid pKYP100, 50 μg of pKYP10 were digested with 50 units of HhaI in 100 μl of a reaction solution containing 10 mM Tris-HCl (pH 7.5), 7 mM MgCl$_2$ and 6 mM 2-mercaptoethanol at 37°C for 2 hours. After digestion with HhaI, a DNA fragment of about 180 bp containing the trp promoter was purified by 5% polyacrylamide gel electrophoresis [A. M. Maxam *et al.*: Proc. Natl. Acad. Sci. *74*, 560 (1977), referred to as PAGE hereinafter]. In the purification step, two DNA fragments other than the desired DNA were obtained because of incomplete purification by PAGE. The three purified DNA fragments (total amount: about 4 μg) were allowed to react with 8 units of *Escherichia coli* DNA polymerase I·Klenow fragment in 30 μl of a reaction solution containing 50 mM Tris-HCl (pH 7.6), 7 mM MgCl$_2$, 10 mM 2-mercaptoethanol, 0.25 mM dATP, 0.25 mM dCTP, 0.25 mM dGTP and 0.25 mM dTTP at 15°C for 2 hours. By the reaction, the 3'-protruding end formed by HhaI digestion was changed to flush end by the 3' → 5' exonuclease activity and 5' → 3' repairing synthesis activity of DNA polymerase I·Klenow fragment. Subsequently, DNA polymerase I·Klenow fragment was inactivated by heating at 72°C for 30 minutes and the NaCl concentration was adjusted to 50 mM with 1 M NaCl. 8 units of HindIII were added and the mixture was allowed to react at 37°C for 2 hours. After digestion with HindIII, the DNA fragment of about 100 bp containing the trp promoter was isolated and purified by PAGE.

Separately, 5 μg of plasmid pBR322 were digested with 8 units of EcoRI in 20 μl of a reaction solution containing 10 mM Tris-HCl (pH 7.5), 50 mM NaCl, 7 mM MgCl$_2$ and 6 mM 2-mercaptoethanol at 37°C for 2 hours. After phenol and chloroform extraction and ethanol precipitation, the precipitated DNA fragment was dissolved in 20 μl of a mixture of 50 mM Tris-HCl (pH 7.6), 7 mM MgCl$_2$, 6 mM 2-mercaptoethanol, 0.25 mM dATP, 0.25 mM dCTP, 0.25 mM dGTP and 0.25 mM dTTP. Then, 8 units of *Escherichia coli* DNA polymerase I·Klenow fragment were added and the mixture was allowed to react at 15°C for 2 hours. The 5'-protruding end formed by EcoRI digestion was changed to flush end by the repairing synthesis activity of DNA polymerase I·Klenow fragment. The DNA polymerase I·Klenow fragment was inactivated by heating at 72°C for 30 minutes and the NaCl concentration was adjusted to 50 mM with 1 M NaCl. 8 units of HindIII were added and the mixture was allowed to react at 37°C for 2 hours. After digestion with HindIII, the larger plasmid DNA fragment of about 4.33 Kb was purified by low-gelling-temperature agarose gel electrophoresis.

About 50 ng of the DNA fragment 3 of about 100 bp containing the trp promoter and obtained above, about 0.2 μg of the DNA fragment 6 of about 4.33 Kb derived from pBR322 and obtained above, and 50 ng of 5'-phosphorylated XhoI linker (pCCTCGAGG, product of Collaborative Research) were ligated with 1 unit of T4DNA ligase in 20 μl of a reaction solution containing 20 mM Tris-HCl (pH 7.6), 10 mM $MgCl_2$, 10 mM dithiothreitol and 0.5 mM ATP at 4°C for 40 hours. *Escherichia coli* HB101 was transformed with the thus obtained recombinant plasmid DNA and plasmid DNAs were isolated and purified from the $Ap^RTc^R$ transformants. These plasmid DNAs were digested with restriction enzymes EcoRI, XhoI, HindIII, HaeIII, ClaI, TaqI (product of BRL) and RsaI (product of New England Biolabs) to select the plasmid 7 wherein the DNA fragment 3 of about 100 bp containing the trp promoter and XhoI linker 4 were cloned. This plasmid was named pKYP100.

Reference Example 3

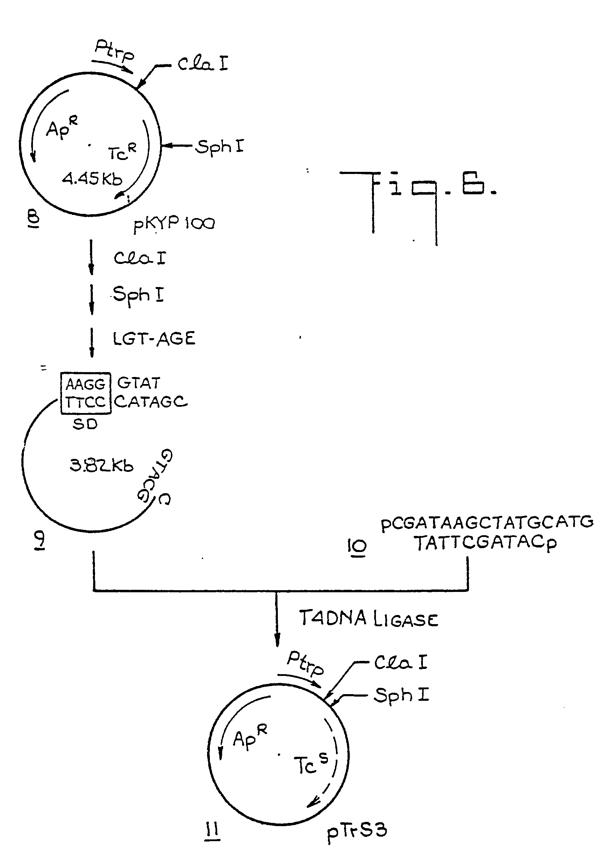

Construction of plasmid vector pTrS3 bearing the initiation codon for translation ATG and SphI cleavage site downstream from the trp promoter and the ribosome binding site (refer to Fig. 6)

Plasmid pTrS3 is obtained from pKYP100 constructed as in Reference Example 2 in the following manner. 5 μg of pKYP100 8 were allowed to react with 5 units of ClaI in 20 μl of a reaction solution containing 10 mM Tris-HCl (pH 7.5), 7 mM $MgCl_2$ and 6 mM 2-mercaptoethanol at 37°C for 2 hours. The reaction was stopped by heating at 65°C for 5 minutes. Then, 2 μl of 100 mM Tris-HCl (pH 7.5), 70 mM $MgCl_2$, 1.0 M NaCl, 60 mM 2-mercaptoethanol, 16 μl of distilled water and 7 units of SphI (product of Boehringer Mannheim GmbH) were added and the mixture was allowed to react at 37°C for 2 hours. The reaction was stopped by heating at 65°C for 5 minutes and the larger plasmid DNA fragment 9 (about 3.82 Kb) was purified by low-gelling-temperature agarose gel electrophoresis.

Separately, two species of oligonucleotides, 5'-CGATAAGCTATGCATG-3' and 5'-CATAGCTTAT-3' were synthesized by the phosphotriester method. The two synthesized oligonucleotides were 5'-phosphorylated and 20 μM each of the oligonucleotides were mixed with 10 mM Tris-HCl (pH 7.5), 100 mM NaCl and 1 mM EDTA. The mixture was incubated at 65°C for 10 minutes, at 37°C for 120 minutes and at room temperature for 120 minutes to anneal them. The two DNA chains were annealed as illustrated below.

pCGATAAGCTATGCATG

TATTCGATACp

Both ends of the resulting DNA fragment can be ligated with the DNA fragment having sticky ends formed by digestion with ClaI or SphI and the ligated DNA has a ClaI cleavage site or SphI cleavage site for reconstruction. The annealed DNA 10 of the two oligonucleotides and the plasmid DNA fragment 9 purified as above were mixed and ligated with T4DNA ligase. That is, 1 pM each of the two oligonucleotides, pCGATAAGCTATGCATG and pCATAGCTTAT were annealed and about 0.15 μg of the purified plasmid DNA fragment 9 was added. Then, 0.5 unit of T4DNA ligase were added and the mixture was allowed to react in 20 μl of a reaction solution containing 20 mM Tris-HCl (pH 7.6), 10 mM $MgCl_2$, 10 mM dithiothreitol and 0.5 mM ATP at 4°C for 16 hours.

*Escherichia coli* HB101 was transformed with the resulting recombinant plasmid DNA. Plasmid DNAs were isolated from the thus obtained transformant resistant to ampicillin and sensitive to tetracycline ($Ap^RTc^S$) and purified. These plasmid DNAs were disgested with EcoRI, XhoI, PstI, ClaI and SphI to recognize the formation of the desired plasmid vector pTrS3 11. It was recognized by the method of Maxam and Gilbert, Proc. Natl. Acad. Sci. 74, 560 (1977) that the base sequence of the DNA between the ClaI site and SphI site of pTrS3 was ATCGATAAGCTATGCATGC. *Escherichia coli* containing pTrS3 was deposited with the Fermentation Research Institute on September 29, 1982 as *Escherichia coli* ITrS—3 FERM P—6735. The deposit was transferred to the international deposit on July 26, 1983 and assigned accession number FERM BP—328.

**Claims**

1. A recombinant plasmid containing an Adult T Cell Leukemia Virus (ATLV) *gag* gene, wherein
   (a) the ATLV *gag* gene is under the control of the tryptophan promoter; and
   (b) the SD sequence is located downstream from said tryptophan promoter in a distance of 6—18 bp upstream from the translation initiation codon ATG.

2. The recombinant plasmid according to claim 1, wherein the distance between the SD sequence and the initiation codon is 13 bp.

3. The recombinant plasmid according to claim 1, which is plasmid pTAG424A carried by E. coli ETAG424A (FERM BP—341) or plasmid pTAG424B carried by E. coli ETAG424B (FERM BP—342).

4. A transformed microorganism which harbours a recombinant plasmid according to any one of claims 1 to 3.

5. The transformed microorganism according to claim 4 which belongs to the genus Escherichia.

6. The transformed microorganism according to claim 4 which is a strain of the species Escherichia coli.

7. A method for the production of a peptide encoded by an ATLV *gag* gene, which comprises culturing in a nutrient medium a microorganism according to any one of claims 4 to 6, accumulating said peptide in the culture and recovering it therefrom.


**Patentansprüche**

1. Rekombinantes Plasmid, enthaltend ein ATLV (Adult T Cell Leukemia Virus)-*gag*-Gen, wobei

(a) das ATLV-*gag*-Gen unter der Kontrolle des Tryptophan-Promotors steht; und

(b) die SD-Sequenz stromabwärts vom Tryptophan-Promotor in einer Entfernung von 6 bis 18 bp stromaufwärts vom Translations-Initiations-Codon ATG liegt.

2. Rekombinantes Plasmid gemäß Anspruch 1, wobei die Entfernung zwischen der SD-Sequenz und dem Initiations-Codon 13 bp beträgt.

3. Rekombinantes Plasmid gemäß Anspruch 1, das das von E. coli ETAG424A (FERM BP—341) getragene Plasmid pTAG424A oder das von E. coli ETAG424B (FERM BP—342) getragene Plasmid pTAG424B ist.

4. Transformierter Mikroorganismus, der ein rekombinantes Plasmid gemäß einem der Ansprüche 1 bis 3 trägt.

5. Transformierter Mikroorganismus gemäß Anspruch 4, der zur Gattung Escherichia gehört.

6. Transformierter Mikroorganismus gemäß Anspruch 4, der ein Stamm der Art Escherichia coli ist.

7. Verfahren zur Herstellung eines von einem ATLV-*gag*-Gen codierten Peptids, bei dem man in einem Nährmedium einen Mikroorganismus gemäß einem der Ansprüche 4 bis 6 züchtet, das Peptid in der Kultur ansammelt und es daraus gewinnt.


**Revendications**

1. Plasmide recombinant contenant un gène *gag* d'un virus de la leucèmie à cellules T de l'adulte (ATLV), dans lequel

(a) le gène *gag* d'ATLV est sous le contrôle du promoteur tryptophane; et

(b) la séquence SD est située en aval dudit promoteur tryptophane à une distance de 6—18 pb en amont du codon ATG d'initiation de traduction.

2. Plasmide recombinant selon la revendication 1, dans lequel la distance entre la séquence SD et le codon d'initiation est de 13 pb.

3. Plasmide recombinant selon la revendication 1 qui est le plasmide pTAG424A porté par E. coli ETAG424A (FERM BP—341) ou le plasmide pTAG424B porté par E. coli ETAG424B (FERM BP—342).

4. Microorganisme transformé qui heberge un plasmide recombinant selon l'une quelconque des revendications 1 à 3.

5. Microorganisme transformé selon la revendication 4, appartenant au genre Escherichia.

6. Microorganisme transformé selon la revendication 4, appartenant à une souche de l'espèce Escherichia coli.

7. Procédé pour la production d'un peptide codé par un gène *gag* d'ATLV, qui comprend la culture, dans un milieu nutritif, d'un microorganisme selon l'une quelconque des revendications 4 à 6, l'accumulation dudit peptide dans la culture et sa récupération à partir de celle-ci.

Fig. 1.

Fig. 2.

Eco RI

pBR322
(4.36Kb)

Ap. Tc

Taq I

Eco RI

pKYP-1
(9.2Kb)

~2.6Kb

Ap.

Ptrp

Tc

Taq I.

D E

Taq I Partial Digestion

Purification Of DNA
Fragment Of 4.36 Kb

Eco RI Complete Digestion

Purification Of DNA
Fragment Of 4.33 Kb

Taq I Partial Digestion

Purification Of DNA
Fragment Of 8.5 Kb

Eco RI Complete Digestion

Purification Of DNA
Fragment Of About 2.6 Kb

Ligation Of DNA Fragments
With T4DNA Ligase

Fig-3.

Eco RI

pKYP-5
(7.0Kb)

Ap

Tc

Ptrp

50

Cla I (Taq I)

Hind III

Fig.4.

## Fig.5.

Fig. 6.